# EUROPEAN PATENT SPECIFICATION

(11) **EP 1 649 768 B1**
(45) Date of publication and mention of the grant of the patent: **16.02.2011**
(21) Application number: 05256535.5
(22) Date of filing: 21.10.2005
(51) Int. Cl.: A41D 13/12, A41D 19/00, A61B 19/04

(54) **Impervious partial sleeve with glove retention**
Undurchlässige Teilhülse mit Handschuhhalterung
Manche partiellement étanche avec des gants de rétention

(30) Priority: 21.10.2004 US 620876
(43) Date of publication of application: 26.04.2006
(73) Proprietor: Allegiance Corporation, McGaw Park, Illinois 60085-6787 (US)
(72) Inventor: Palomo, Joe, Antioch, Illinois 60002 (US); Miller, Joe, El Paso, Texas 79912 (US); Amaya, Fernando, El Paso, Texas 7 (US)
(74) Representative: Dee, Ian Mark

(56) References cited:
- US-A- 4 996 722
- US-A- 5 594 955
- US-A1- 2004 123 367
- US-B1- 6 530 090
- US-B1- 6 638 605

## Description

### FIELD OF THE INVENTION

The present invention relates to protective garments for use with gloves, for example surgical gowns used with surgical gloves.

### BACKGROUND OF THE INVENTION

The practice of wearing of a surgical gown and surgical gloves to protect health care providers from contamination by body fluids is well accepted; however, one risk faced by health care providers is that such protective barrier can become breached during interaction with a patient. With the increase of highly infectious diseases, such as the acquired immune deficiency syndrome (AIDS) and hepatitis, the risk to health care providers of such protective barrier breach has become acute.

Typically, protective garments rely on the barrier properties of the fabrics used in the garments and on the construction and design of the garment. Openings in the garments that are located in positions subject to contact with body fluids pose a particular risk of contamination.

In the medical industry, the protective gloves are pulled up and over the sleeve of a gown or garment to reduce the openings in the garment; however, the interface between the glove and the protective garment pose a particular risk of contamination. For example, a common issue with surgical gloves is glove "roll-down" or slippage between the interior side of the glove and the surgical gown sleeve. When the glove rolls down on the sleeve, the wearer is at greater risk of exposure to body fluids.

An additional problem associated with the use of surgical gloves is a phenomenon known as "channeling". Channeling occurs when the sleeve of the gown is bunched up under the glove as a result of pulling and rolling the glove up over the cuff and sleeve. Channels may develop along the wrist which can become accessible to body fluids running down the outside of the sleeve of the gown. Body fluids can work down along the channels between the outer surface of the gown and inner surface of the surgical glove. These fluids may then contaminate the gown cuff, which lies directly against the wrist or forearm, particularly if the cuff is absorbent or fluid pervious.

Several attempts have been made to minimize exposure at the glove and gown interface. For example, surgeons commonly use adhesive tape wrapped around the glove portion extending over the gown sleeve to prevent channeling and roll down of the glove; however, common adhesives utilized in tapes are subject to degradation by water and body fluids. It is also common to stretch a rubber band around the glove and sleeve; however, it is difficult to adjust or vary the pressure exerted by the rubber band other than by having a variety of rubber bands of different sizes and tensions available for use.

More recently, attempts have been made to address the exposure at the glove and gown interface structurally in the garments. One such attempt involves sealing together the interface of the garment and gloves. The seal is produced by narrowing the diameter of the distal end of the gown over which the protective glove is placed. The junction of the garment and glove is then sealed with a liquid adhesive. This attempt produces a costly integrated garment that is cumbersome and difficult to use in practice; for example, it is very difficult to match the appropriate size of the gown for a given user with the right glove size.

Another such attempt utilizes a raised band disposed on the sleeves of the gown. The raised band attempts to inhibit a glove pulled there over from rolling or sliding. Incorporating a raised band to the sleeves of the gown adds to the complexity and costs of manufacturing the gown and has proven less than optimal. A related attempt involves adding an adhesive to the sleeve to secure the glove thereon. As previously noted, adhesives are subject to degradation by water and body fluids. In addition, the use of adhesives may require a release liner that would be cumbersome to remove prior to pulling on the glove. In addition the glove may stick to the adhesive before it is positioned properly and tear the glove. Other contaminates could be transferred to the adhesive from any incidental contact.

US 6,638,605 B1 discloses disposable surgical drapes and gowns which exhibit desirable softness, foldability, absorbency and breathability characteristics which comprise film layers and non-woven web layers which are secured together by spaced apart unbounded adhesive clusters. Each adhesive cluster comprises a plurality of discrete spaced apart droplets of adhesive securing the film layer and the web layer together. The low adhesive content and flexible bounding methods results in the improved characteristics of the laminate material.

US 4,996,722 discloses a surgical garment having a sleeve reinforcement that reduces the fluid strike through. The sleeve seam is sewn without the sleeve reinforcement, and subsequently the edges of the sleeve reinforcement are glued in an overlapping manner over the sleeve seam in order to present ingress of liquid in use.

US 2004/0123367 discloses protective garments having an outer surface which is provided with a low surface tension liquid blocking material in a continuous unbroken band, region or combination of such, for blocking the wicking of liquid that is contained on the outside surface of the gannet.

US 6,530,090.B1 disclose a protective garment such as a surgical gown which has a circumferentially extending raised band on the sleeve at a distance from the end of the sleeve The band has a raised profile such that an end of a glove pulled over is inhibited from rolling or sliding back over the band and down the sleeves.

Thus, what would be beneficial is an improved interface between a glove and a sleeve of a protective garment. Such improved interface should be easily incorporated with the protective garment and economically cost effective to implement and use.

### SUMMARY OF THE INVENTION

According to the invention, there is provided a protective garment according to claim 1, a sleeve in a garment according to claim 10 and a method of manufacturing a protective garment according to claim 18.

A protective garment in accordance with the principles of the present invention provides an improved interface between a glove and sleeve of the protective garment. A protective garment in accordance with the principles of the present invention is easily incorporated with the protective garment and economically cost effective to implement and use. A protective garment in accordance with the principles of the present invention provides an impervious partial sleeve having a surface exhibiting non-adhesive friction when in contact with dry glove materials.

### BRIEF DESCRIPTION OF THE DRAWINGS

Figure 1 is a perspective view of a surgical gown in accordance with the present invention.

Figure 2 is a close-up perspective view of a sleeve portion in accordance with the present invention.

Figure 3 is a schematic of a blank or pattern for making a sleeve portion in accordance with the present invention.

### DETAILED DESCRIPTION OF THE PREFERRED EMBODIMENTS

An improved protective garment in accordance with the principles of the present invention comprises an improved interface between a glove and sleeve of the protective garment. In one embodiment of the present invention, an improved interface is provided between a glove and a sleeve of a protective garment. In one embodiment of the present invention, an improved interface is provided between a surgical glove and a sleeve of a surgical gown. In one embodiment of the present invention, an impervious partial sleeve is designed to augment an unreinforced or fabric reinforced single use surgical gown sleeve from above a cuff hem to about mid-forearm, thus providing an impervious layer underneath the glove cuff. In addition, the impervious partial sleeve comprises a surface exhibiting friction when in contact with dry glove materials that will help hold the glove up on the forearm. Thus, an improved protective garment in accordance with the principles of the present invention is an improvement over prior art unreinforced gowns as such prior art gowns that typically consist of a spunlaced or spunbond/meltblown/spunbond (SMS) non-woven fabric throughout the body and sleeves. These fabrics are typically not impervious and do not help to provide significant friction against the inside cuff of the glove.

In one embodiment, the impervious partial sleeve can be a two-layer structure comprising a non-woven fabric and a film. Typical non-woven fabrics examples that can be used include, but are not limited to carded polyester, spun laced wood pulp and polyester, spun bond polyolefin, and SMS polyolefin. Typical film examples that can be used include, but are not limited to polyethylene, ethylene-vinyl-acetate copolymer (EVA), molten ethylene methyl acrylate (EMA), polyester co-polymers, polyurethanes, and metallocene polyolefins. In one embodiment, the film can be Hytrel® co-polyester elastomer available from E. I. DuPont De Nemours & Company, 1007 Market Street, Wilmington Delaware 19898 U.S.A. Multilayer films that may incorporate some of the example polymers listed but put the friction layer on the top layer for contact with the glove may also be used. The film and non-woven fabric can be laminated for example by adhesive, thermal bonding or by extrusion coating.

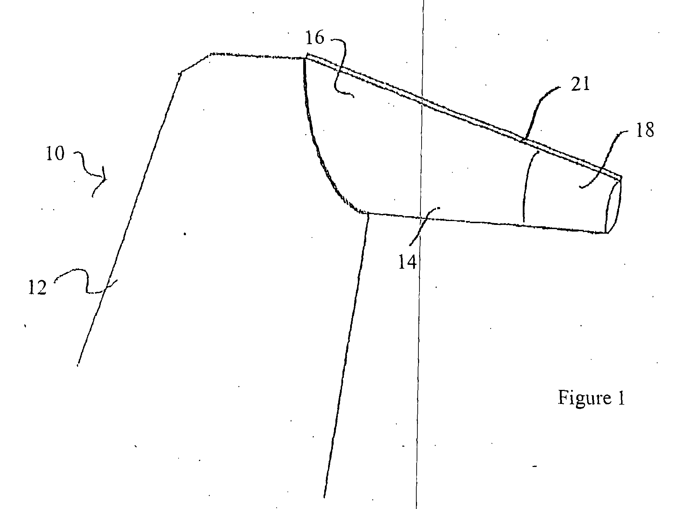

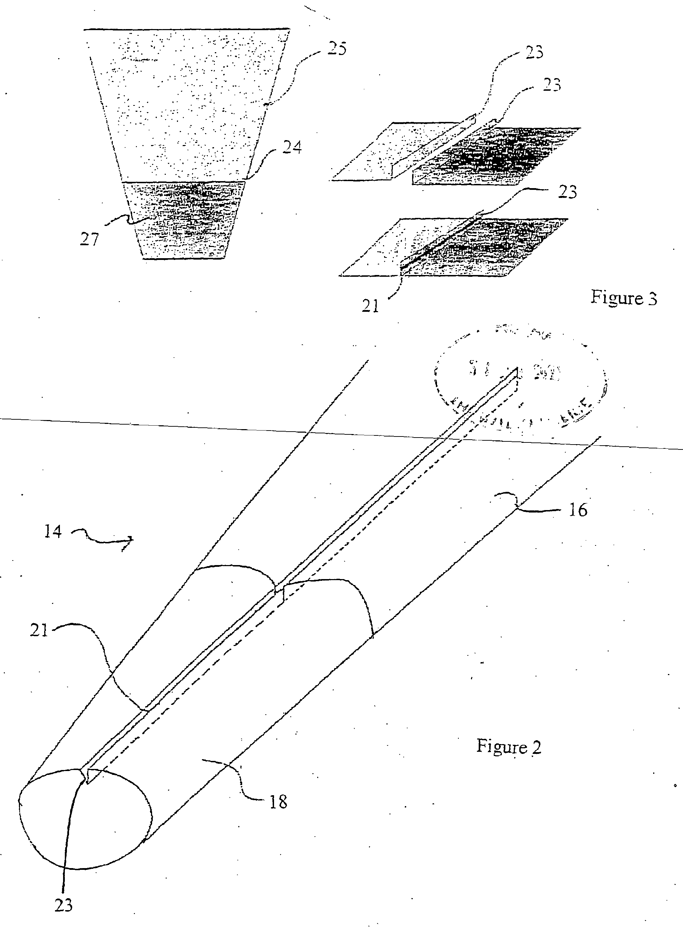

Referring to Figure 1, a perspective view of a portion of a surgical gown in accordance with the present invention is seen. The surgical gown 10 includes a body portion 12 and a sleeve portion 14. The surgical gown 10 including the sleeve portion 14 can comprise a non-woven fabric such as, for example, spun laced wood pulp and polyester, spun bond polyolefin, and SMS polyolefm. Referring to Figure 2, a close-up perspective view of a sleeve portion 14 in accordance with the present invention is seen.

In one embodiment of the present invention, the sleeve portion 14 comprises an upper sleeve 16 and a lower sleeve 18.

In accordance with the present invention, the lower sleeve 18 comprises an impervious partial sleeve that augments the sleeve portion 14 from above a cuff hem to about mid-forearm, thus providing an impervious layer underneath the glove cuff. In further accordance with the present invention, the lower sleeve 18 comprises a surface exhibiting friction when in contact with dry glove materials that will help to hold the glove up on the forearm. As previously described, the lower sleeve 18 can comprise a film adhered to the non-woven fabric. In one embodiment, the film can be Hytrel® co-polyester elastomer available from E. I. DuPont De Nemours & Company. The film and non-woven fabric can be laminated for example by adhesive, thermal bonding or by extrusion coating.

Referring to Figure 3, a schematic of a blank or pattern 20 for making a sleeve portion 14 in accordance with the present invention is seen. The blank 20 comprises an upper sleeve blank 25 and a lower sleeve blank 27, both in the form of a trapezoid. To make the present invention, the lower sleeve blank 27 can be attached to the non-woven fabric of the upper sleeve blank 25 by heat sealing. A seam 24 is formed where the edges of the upper sleeve blank 25 and the lower sleeve blank 27 overlap and the non-woven fabric can be bonded to the lower sleeve blank 27. Other means can be used to seal the lower sleeve blank 27 to the upper sleeve blank 25 such as for example adhesive or ultrasonic sealing.

After the lower sleeve blank 27 is attached to the upper sleeve blank 25, the whole length of the sleeve blank can be heat sealed to form the sleeve portion 14. The length of the sleeve portion 14 can be closed into a tube by for example mechanically bonding the outer film layer to itself to form a seam 21 such as for example by hot melt glue. This will yield a sealed ridge 23 on the inside of the sleeve portion 14. Other means can be used to seal the sleeve portion 14 such as for example adhesive or ultrasonic sealing. In one embodiment, a cuff (not shown) can then be attached to the end of the lower sleeve blank 27. Both sleeve portions 14 of the gown 10 would have this film of the lower sleeve blank 27. In an alternative embodiment, the film can be adhered to the lower portion of a full nonwoven sleeve blank.

A mechanical bond can be achieved with for example thermal, chemical or adhesive bonds. The thermal bond is a method that applies heat to either one or both surfaces with heat from a source, pressure and/or vibration until one or both materials reach an emulsified state, then mechanically pressing the materials together forming a seal. The chemical process is a procedure in which a chemical agent is added between both edges of the materials creating a chemical reaction that emulsifies the material, and then mechanically pressing materials together until the chemical reaction is completed. The mechanical process is a method that puts adhesive between the materials. Pressure is used to bond the layers together and form a seal. Pressing the materials against the adhesive, the material adheres to the adhesive forming a seal. The adhesive is applied for example with an applicator, tape substrates or tapes. The use of a film outer layer and the mechanically bonded seam will allow an impervious claim on the fabric and the seam based on the standard test method of ASTM International F1671, Standard Test Method for Resistance of Materials Used in Protective Clothing to Penetration by Blood-Borne Pathogens Using Phi-X174 Bacteriophage Penetration as a test system.

The following are examples are presented for the purposes of explanation and not to narrow the scope of the present invention.

### Example 1:

In this example, a breathable impervious (BI) surgical gown was made in accordance with the principles of the present invention utilizing as the film Hytrel® co-polyester elastomer available from E. I. DuPont De Nemours & Company. The film was adhered to the non-woven fabric by adhesive. The lower sleeve blank was attached to the non-woven fabric of the upper sleeve blank by adhesive, forming a seam where the edges of the upper sleeve blank and the lower sleeve blank overlap. The length of the sleeve blank was then adhered with adhesive to form a tube.

Using a sample size of 10 of the BI surgical gowns, the coefficient of friction was tested between the surgical gowns and two dry gloves utilizing the INDA Standard Test (IST) 140.1 (01) (Static and Kinetic Coefficient of Friction ofNonwoven Fabrics method. A constant rate of motion machine was used, such as a Zwick tester available from Zwick USA, 1620 Cobb International Blvd., Suite 1, Kennesaw, Georgia 30152. The test used a moving sled with a stationary plane to determine the starting (static) and sliding (kinetic) friction between two substrates. In this case the glove cuff was wrapped around the sled exposing the inside surface, which contacts the gown sleeve. The gown sleeve fabric was attached to the plane so that the sled wrapped with the glove would slide over it. The two dry gloves were the Biogel gloves (Catalog Number 30475) available from Regent Medical, 3585 Engineering Drive, Suite 250, Norcross, Georgia 30092 U.S.A. and the Protegrity gloves (Catalogue Number 2D72N41) available from Cardinal Healthcare, 1430 Waukegan Road, McGaw Park, Illinois 60085 U.S.A.

The average static coefficient of friction between the BI surgical gown of the present invention and the Biogel glove was 0.64 (with a standard deviation of 0.13) while the average static coefficient of friction between the BI surgical gown of the present invention and the Protegrity glove was 0.71 (with a standard deviation of 0.15).

### Example 2:

In this example, again using a sample size of 10, the coefficient of friction was tested utilizing the same method between an Astound surgical gown (Catalogue Number 9545) available from Cardinal Healthcare and the Biogel gloves (Catalog Number 30475) and the Protegrity gloves (Catalogue Number 2D72N41). The average static coefficient of friction between the Astound surgical gown and the Biogel glove was 0.07 (with a standard deviation of 0.03) while the average static coefficient of friction between the Astound surgical gown and the Protegrity glove was 0.17 (with a standard deviation of 0.05).

### Example 3:

In this example, again using a sample size of 10, the coefficient of friction was tested utilizing the same method between an KC Ultra surgical gown (Catalogue Number 95121) available from Kimberly-Clark, 1400 Holcomb Bridge Road, Roswell, Georgia 30076 U.S.A. and the Biogel gloves (Catalog Number 30475) and the Protegrity gloves (Catalogue Number 2D72N41). The average static coefficient of friction between the KC Ultra surgical gown and the Biogel glove was 0.09 (with a standard deviation of 0.03) while the average static coefficient of friction between the KC Ultra surgical gown and the Protegrity glove was 0.17 (with a standard deviation of 0.06).

### Example 4:

In this example, again using a sample size of 10, the coefficient of friction was tested utilizing the same method between an Impervious Astound surgical gown (Catalogue Number 9040) available from Cardinal Healthcare and the Biogel gloves (Catalog Number 30475) and the Protegrity gloves (Catalogue Number 2D72N41). The average static coefficient of friction between the Impervious Astound surgical gown and the Biogel glove was 0.18 (with a standard deviation of 0.05) while the average static coefficient of friction between the Impervious Astound surgical gown and the Protegrity glove was 0.20 (with a standard deviation of 0.06).

### Example 5:

In this example, again using a sample size of 10, the coefficient of friction was tested utilizing the same method between an Optima surgical gown (Catalogue Number 9507CE) available from Cardinal Healthcare and the Biogel gloves (Catalog Number 30475) and the Protegrity gloves (Catalogue Number 2D72N41). The average static coefficient of friction between the Optima surgical gown and the Biogel glove was 0.10 (with a standard deviation of 0.01) while the average static coefficient of friction between the Optima surgical gown and the Protegrity glove was 0.29 (with a standard deviation of 0.12).

It is seen that a surgical gown in accordance with the principles of the present invention marked an increase in the coefficient of friction with dry surgical gloves. Thus, an improved surgical gown in accordance with the principles of the present invention provides an improved interface between a glove and sleeve of a protective garment. An improved surgical gown in accordance with the principles of the present invention is easily incorporated with the protective garment and economically cost effective to implement and practice.

While the invention has been described with specific embodiments, other alternatives, modifications, and variations will be apparent to those skilled in the art. Accordingly, it will be intended to include all such alternatives, modifications and variations set forth within the scope of the appended claims.

## Claims

1. A protective garment (10) comprising:
at least a portion (18) of the outer surface a sleeve (14) having a surface applied thereon to increase non-adhesive friction when in contact with dry glove materials, wherein a raised band is not disposed on the sleeve (14).

2. The protective garment (10) of claim 1 wherein the surface applied to at least a portion (18) of the outer surface of sleeve (14) is impervious.

3. The protective garment (10) of claim 1 wherein the surface exhibiting non-adhesive friction comprises the outer surface of the sleeve (14) from above a cuff to about mid-foreann.

4. The protective garment (10) of claim 1 wherein the garment (10) comprises an unreinforced single use surgical gown.

5. The protective garment (10) of claim 1 wherein the garment (10) comprises a fabric reinforced single use surgical gown.

6. The protective garment (10) of claim 1 wherein the surface exhibiting non-adhesive friction comprises a two-layer structure comprising a non-woven fabric and a film.

7. The protective garment (10) of claim 6 wherein the film is multilayer.

8. The protective garment (10) of claim 6 wherein the film is selected from the group comprising co-polyester elastomer, polyurethane, polyethylene, ethylene-vinyl-acetate copolymer (EVA), molten ethylene methyl acrylate (EMA), metallocene polyolefins, and combinations thereof.

9. The protective garment (10) of claim 6 further wherein the film comprises Hytrel^{®} co-polyester elastomer.

10. A sleeve (14) in a garment (10) comprising:
a surface applied to at least a portion (18) of the outer surface of a sleeve (14) that is imperious and increases non-adhesive friction when in contact with dry glove materials.

11. The sleeve (14) of claim 9 wherein the surface exhibiting non-adhesive friction comprises the outer surface of the sleeve (14) from above a cuff to about mid-forearm.

12. The sleeve (14) of claim 10 wherein the garment (10) comprises an unreinforced single use surgical gown.

13. The sleeve (14) of claim 10 wherein the garment (10) comprises a fabric reinforced single use surgical gown.

14. The sleeve (14) of claim 10 wherein the surface exhibiting non-adhesive friction comprises a two-layer structure comprising a non-woven fabric and a film.

15. The sleeve (14) of claim 14 wherein the film is multilayer.

16. The sleeve (14) of claim 14 wherein the film is selected from the group comprising co-polyester Elastomer, polyurethane, polyethylene, ethylene-vinyl-acetate copolymer (EVA), molten ethylene methyl acrylate (EMA), metallocene polyolefins, and combinations thereof.

17. The, sleeve (14) of claim 14 wherein the film comprises Hytrel^{®} co-polyester elastomer.

18. A method of manufacturing a protective garment (10) comprising:
adhering to at least a portion (18) of the outer surface of a sleeve (14) a surface that increases non-adhesive friction when in contact with dry glove materials, wherein a raised band is not disposed on the sleeve (14).

19. The method of manufacturing of claim 18 comprising adhering the surface exhibiting non-adhesive friction to the outer surface of the sleeve (14) from above a cuff to about mid-forearm.

20. The method of manufacturing of claim 18 wherein the garment (10) is an unreinforced single use surgical gown.

21. The method of manufacturing of claim 18 wherein the garment (10) is a fabric reinforced single use surgical gown.

22. The method of manufacturing of claim 18 comprising adhering a film exhibiting non-adhesive friction to a non-woven fabric.

23. The method of manufacturing of claim 22 wherein the film is selected from the group comprising co-polyester elastomer, polyethylene ethylene-vinyl-acetate copolymer (EVA.), molten ethylene methyl acrylate (EMA), metallocene polyolefins, and combinations thereof.

24. The method of manufacturing of claim 22 wherein the film comprises Hytrel^{®} co-polyester elastomer.

25. A method of manufacturing according to any of claims 18 to 24 wherein the surface that increases mon-adhessive friction when in contact with dry glove materials is impervious.

## Patentansprüche

1. Schutzbekleidung (10), welche enthält:
zumindest einen Abschnitt (18) von der Aussenfläche von einer Hülse (14), welche eine daran angelegte Fläche hat, um eine nicht-haftende Reibung zu erhöhen, wenn sie mit trockenen Handschuh-Materialien in Kontakt steht, wobei kein erhöhtes Band auf der Hülse angeordnet ist.

2. Schutzbekleidung (10) nach Anspruch 1, bei welcher die Fläche, welche an zumindest einen Abschnitt (18) von der Aussenfläche von der Hülse (14) angeordnet ist, undurchlässig ist.

3. Schutzbekleidung (10) nach Anspruch 1, bei welcher die Fläche, welche die nicht-haftende Reibung darlegt, die Aussenfläche von der Hülse (14) von oberhalb einer Stulpe bis etwa zur Mitte des Unterarms enthält.

4. Schutzbekleidung (10) nach Anspruch 1, wobei die Schutzbekleidung (10) einen nicht-verstärkten Einweg-Operationskittel enthält.

5. Schutzbekleidung (10) nach Anspruch 1, wobei die Schutzbekleidung (10) einen gewebeverstärkten Einweg-Operationskittel enthält.

6. Schutzbekleidung (10) nach Anspruch 1, bei welcher die Fläche, welche die nicht-haftende Reibung darlegt, einen doppelschichtigen Aufbau enthält, welcher ein nicht-verwobenes Gewebe und eine Folie enthält.

7. Schutzbekleidung (10) nach Anspruch 6, bei welcher die Folie mehrschichtig ist.

8. Schutzbekleidung (10) nach Anspruch 6, bei welcher die Folie aus der Gruppe ausgewählt ist, welche ein Co-Polyester Elastomer, Polyurethan, Polyethylen, Ethylen-Vinyl-Acetat Co-Polymer (EVA), geschmolzenes Ethylen-Methyl-Akrylat (EMA), Metallocene-Polyolefin und Kombinationen daraus enthält.

9. Schutzbekleidung (10) nach Anspruch 6, bei welcher der Film Hytrel® Co-Polyester Elastomer enthält.

10. Hülse (14) in einer Bekleidung (10), welche enthält:
eine Fläche, welche an zumindest einen Abschnitt (18) der Aussenfläche von einer Hülse (14) angelegt ist, welche undurchlässig ist und eine nicht-haftende Reibung erhöht, wenn sie mit trockenen Handschuh-Materialien in Kontakt steht.

11. Hülse (14) nach Anspruch 9, bei welcher die Fläche, welche die nicht-haftende Reibung darlegt, die Aussenfläche von der Hülse (14) von oberhalb einer Stulpe bis etwa zur Mitte des Unterarms enthält.

12. Hülse (14) nach Anspruch 10, wobei die Bekleidung (10) einen nicht-verstärkten Einweg-Operationskittel enthält.

13. Hülse (14) nach Anspruch 10, wobei die Bekleidung (10) einen gewebevcrstärkten Einweg-Operationskittel enthält.

14. Hülse (14) nach Anspruch 10, bei welcher die Fläche, welche die nicht-haftende Reibung darlegt, einen doppelschichtigen Aufbau enthält, welcher ein nicht-verwobenes Gewebe und eine Folie enthält.

15. Hülse (14) nach Anspruch 14, bei welcher die Folie mehrschichtig ist.

16. Hülse (14) nach Anspruch 14, bei welcher die Folie aus der Gruppe ausgewählt ist, welche ein Co-Polyester Elastomer, Polyurethan, Polyethylen, Ethylen-Vinyl-Acetat Co-Polymer (EVA), geschmolzenes Ethylen-Methyl-Akrylat (EMA), Metallocene-Polyolefin und Kombinationen daraus enthält.

17. Hülse (14) nach Anspruch 14, bei welcher der Film Hytrel® Co-Polyester Elastomer enthält.

18. Verfahren zur Herstellung von einer Schutzbekleidung (10), welches enthält:
Anhaften an zumindest einen Abschnitt (18) von der Aussenfläche von einer Hülse (14) von einer Fläche, welche eine nicht-hallende Reibung erhöht, wenn sie mit trockenen Handschuh-Materialien in Kontakt steht, wobei kein erhöhtes Band auf der Hülse (14) angeordnet ist.

19. Verfahren zur Herstellung nach Anspruch 18, welches das Anhaften der Fläche, welche die nicht-haftende Reibung darlegt, an die Aussenfläche von der Hülse (14) von oberhalb einer Stulpe bis etwa zur Mitte des Unterarms enthält.

20. Verfahren zur Herstellung nach Anspruch 18, wobei die Bekleidung (10) ein nicht-verstärkter Einweg-Operationskittel ist.

21. Verfahren zur Herstellung nach Anspruch 18, wobei die Bekleidung (10) ein gewebeverstärkter Finweg-Operationskittel ist.

22. Verfahren zur Herstellung nach Anspruch 18, welches das Anhaften von einer Folie, welche die nicht-haftende Reibung darlegt, an ein nicht-verwobenes Gewebe enthält.

23. Verfahren zur Herstellung nach Anspruch 22, bei welchem die Folie aus der Gruppe ausgewählt ist, welche ein Co-Polyester Elastomer, Polyurethan, Polyethylen, Ethylen-Vinyl-Acetat Co-Polymer (EVA), geschmolzenes Ethylen-Methyl-Akrylat (EMA), Metallocene-Polyolefin und Kombinationen daraus enthält.

24. Verfahren zur Herstellung nach Anspruch 22, bei welchem der Film ein Hytrel® Co-Polyester Elastomer enthält.

25. Verfahren zur Herstellung nach einem der Ansprüche 18 bis 24, bei welchem die Fläche, welche die nicht-haftende Reibung erhöht, wenn sie mit trockenen Handschuh-Materialien in Kontakt ist, undurchlässig ist.

## Revendications

1. Vêtement protecteur (10) comprenant :
au moins une portion (18) de la surface externe d'une manche (14) sur laquelle est appliquée une surface pour augmenter le frottement non adhésif une fois en contact avec des matériaux de gant sec, dans laquelle un nerf n'est pas disposé sur la manche (14).

2. Vêtement protecteur (10) selon la revendication 1, dans lequel la surface appliquée à au moins une portion (18) de la surface externe de la manche (14) est imperméable.

3. Vêtement protecteur (10) selon la revendication 1, dans lequel la surface présentant un frottement non adhésif comprend la surface externe de la manche (14) allant au-delà d'un poignet jusqu'à environ la moitié de l'avant-bras.

4. Vêtement protecteur (10) selon la revendication 1, dans lequel le vêtement (10) comprend une blouse chirurgicale à usage unique non renforcée.

5. Vêtement protecteur (10) selon la revendication 1, dans lequel le vêtement (10) comprend une blouse chirurgicale à usage unique renforcée de tissu.

6. Vêtement protecteur (10) selon la revendication 1, dans lequel la surface présentant un frottement non adhésif comprend une structure à deux couches comprenant un tissu non tissé et un film.

7. Vêtement protecteur (10) selon la revendication 6, dans lequel le film est une multicouche.

8. Vêtement protecteur (10) selon la revendication 6, dans lequel le film est choisi dans le groupe consistant en un élastomère de copoly(ester), le poly(uréthane), le poly(éthylène), un copolymère d'éthylène-acétate de vinyle (EVA), l'échylène-acrylate de méthyle (EMA) fondu, les poly(oléfines) métallocène, et leurs combinaisons.

9. Vêtement protecteur (10) selon la revendication 6, dans lequel le film comprend un élastomère de copoly(ester) Hytrel^{®}.

10. Manche (14) dans un vêtement (10) comprenant :
une surface appliquée à au moins une portion (18) de la surface externe d'une manche (14) qui est imperméable et augmente le frottement non adhésif une fois en contact avec des matériaux de gant sec.

11. Manche (14) selon la revendication 9, dans laquelle la surface présentant un frottement non adhésif comprend la surface externe de la manche (14) allant depuis au-delà d'un poignet jusqu'à environ la moitié de l'avant-bras.

12. Manche (14) selon la revendication 10, dans laquelle le vêtement (10) comprend une blouse chirurgicale à usage unique non renforcée.

13. Manche (14) selon la revendication 10, dans laquelle le vêtement (10) comprend une blouse chirurgicale à usage unique renforcée de tissu.

14. Manche (14) selon la revendication 10, dans laquelle la surface présentant un frottement non adhésif comprend une structure à deux couches comprenant un tissu non tissé et un film.

15. Manche (14) selon la revendication 14, dans laquelle le film est multicouche.

16. Manche (14) selon la revendication 14, dans laquelle le film est choisi dans le groupe consistant en un élastomère de copoly(ester), le poly(uréthane), le poly(éthylène), un copolymère d'éthylène-acétate de vinyle (EVA), l'éthylène-acrylate de méthyle (EMA) fondu, les poly(oléfines) métallocène, et leurs combinaisons.

17. Manche (14) selon la revendication 14, dans laquelle le film comprend un élastomère de copoly(ester) Hytrel^{®}.

18. Procédé de fabrication d'un vêtement protecteur (10) comprenant l'étape consistant à :
faire adhérer à au moins une portion (18) de la surface externe d'une manche (14) une surface qui augmente le frottement non adhésif une fois en contact avec des matériaux de gant sec, où un nerf n'est pas disposé sur la manche (14).

19. Procédé de fabrication selon la revendication 18, comprenant l'étape consistant à faire adhérer la surface présentant le frottement non adhésif à la surface externe de la manche (14) allant depuis au-delà d'un poignet jusqu'à environ la moitié de l'avant-bras.

20. Procédé de fabrication selon la revendication 18, dans lequel le vêtement (10) est une blouse chirurgicale à usage unique non renforcée.

21. Procédé de fabrication selon la revendication 18, dans lequel le vêtement (10) est une blouse chirurgicale à usage unique renforcée de tissu.

22. Procédé de fabrication selon la revendication 18, comprenant l'étape consistant à faire adhérer un film présentant un frottement non adhésif à un tissu non tissé.

23. Procédé de fabrication selon la revendication 22, dans lequel le film est choisi dans le groupe comprenant un élastomère de copoly(ester), le poly(éthylène), un copolymère d'éthylène-acétate de vinyle (EVA), l'éthylène-acrylate de méthyle (EMA) fondu, les poly(oléfines) métallocène, et leurs combinaisons.

24. Procédé de fabrication selon la revendication 22, dans lequel le film comprend un élastomère de copoly(ester) Hytrel^{®}.

25. Procédé de fabrication selon l'une quelconque des revendications 18 à 24, dans lequel la surface qui augmente le frottement non adhésif une fois en contact avec des matériaux de gant sec est imperméable.
